# EUROPEAN PATENT APPLICATION

(11) **EP 2 524 707 A1**
(43) Date of publication of application: **21.11.2012**
(21) Application number: 11166273.0
(22) Date of filing: 16.05.2011
(51) Int. Cl.: A61L 27/02, A61L 27/12, A61L 27/24

(54) **Injectible, biocompatible synthetic bone growth composition**

(71) Applicant: Beijing Allgens Medical Science & Technology Co., Ltd., Beijing 100085 (CN)
(72) Inventor: Hu, Eric G,, New Jersey, NJ 07920 (US); Hu, Kun, 100085, Haidian District (CN); Chenguang, Liu, 100085, Haidian District (CN); Zonggang, Chen, 100085, Haidian District (CN); Fuzhai, Cui, 100085, Haidian District (CN)
(74) Representative: Clegg, Richard Ian

(57) **Abstract**

An injectible, biocompatible synthetic bone growth composition comprising a mineralized collagen and a calcium sulfate component. The composition is formed into injectible formulation that may be provided at the site of a skeletal defect via minimally invasive manner. An osteoinductive component may be further added, either before or after forming the unitary article. The composition may be formulated as a paste or putty and facilitates bone growth and/or repair.

## Description

### FIELD OF THE INVENTION

The invention relates generally to an injectible composition comprising biocompatible synthetic mineralized collagen component and a calcium sulfate component, a method of making the composition, and a use of the composition in promoting bone growth and/or repair.

### BACKGROUND OF THE INVENTION

Autologous bone grafts are the gold standard for restoring skeletal defects because they provide both a natural tissue scaffold and osteoinductive growth factors. Allogenic grafts may also be used, such as demineralized bone matrices. Because autogenic and allogenic sources of human bone are limited and may be expensive or painful to obtain, the use of substitute materials is preferred. Numerous synthetic or modified natural materials have been experimentally evaluated as alternative delivery vehicles, and include but are not limited to products containing hydroxyapatites, tricalcium phosphates, aliphatic polyesters (poly(lactic) acids (PLA), poly(glycolic)acids (PGA), polycaprolactone (PCL), cancellous bone allografts, human fibrin, plaster of Paris, apatite, wollastonite (calcium silicate), glass, ceramics, titanium, devitalized bone matrix, non-collagenous proteins, collagen and autolyzed antigen extracted allogenic bone. However, most of these synthetic or modified natural materials have yet to result in delivery vehicles having osteoinductivity comparable to autograft or allograft bone sources, or they also need the surgeon to perform open surgeries to implant these bone grafts.

Thus, alternate products are desirable to provide not only bone repair but also in a minimally invasive manner, so that to minimize the impact of surgery on patients.

### BRIEF SUMMARY OF THE INVENTION

The term "Minimally Invasive Bone Grafting" refers to new techniques of bone grafting in which the grafting procedure can be done using injection through a needle, avoiding the need for a surgical incision.

The material is meant to be injected by cannula into hard to reach sites in order to provide a bone graft option for precise placement into difficult-to-reach surgical sites, at the same time forming scaffolding for bone marrow formation and osteoprogenitor cell growth.

All methods of bone grafting involve adding some material to the specific site where bone is needed as a means of stimulating a new or more effective bone healing response. Now, minimally invasive bone grafting is available, meaning that the grafting can be performed with a needle, without a surgical incision.

Biocompatible compositions that comprise bone growth particles, a method of making the compositions, and uses of the compositions in promoting bone growth are disclosed. One embodiment is a bone growth-promoting composition comprising mineralized collagen and calcium sulfate that can be formulated as a paste or putty. The compositions and methods facilitate skeletal regeneration in a minimally invasive manner and provide a scaffold for new bone growth.

The compositions may be formulated as pastes or putties. This provides ease of use and economy of product manufacture. Pastes and putties are soft masses with physical consistencies between a liquid and a solid. Pastes and putties are desirable for surgical bone repair as they can be more easily delivered to difficult surgical sites and molded in site into desired shapes. These products are desirable for the reconstruction of skeletal defects, e.g., in spine, dental, and/or other orthopedic surgeries. They may be used as a substitute for autologous bone grafts or may be used in conjunction with autologous bone grafts.

One embodiment is a biocompatible synthetic bone growth composition comprising a particulate composite of a mineralized collagen component and a calcium sulfate component. In the mineralized collagen component, the collagen component may be insoluble collagen (e.g., crosslinked collagen or porous particles) The calcium phosphate component may be acidic calcium phosphate, such as monocalcium phosphate [Ca(H₂PO₄)₂], calcium hydrogen phosphate dihydrate [CaHPO₄ 2H₂O], anhydrous calcium hydrogen phosphate [CaHPO_{4]}, partially dehydrated calcium hydrogen phosphate [CaHPO₄xH₂O, where x is between and includes 0 and 2] and/or calcium pyrophosphate [2CaO.P₂O₅]. The calcium sulfate might be calcium sulfate hemihydrates [CaS0_{4.1/2}H₂O], partially dehydrate calcium sulfate [CaSO₄xH₂O, where x is between and includes 0 and 2].

Another embodiment is a process for producing a bone growth composition. A collagen component is combined with a calcium phosphate component to produce a mineralized collagen component. The mineralized collagen component may be prepared as a collagen gel, which may be frozen and lyophilized into a product referred to as a sponge. Particles of the mineralized collagen component (e.g. sponge) may be prepared by grinding, milling, chopping and/or molding the mineralized collagen component. The particulate composition may be packaged as a kit that may include a device (e.g., container) for mixing the particles with a fluid. An osteoinductive component may be added, either before or after forming the particles.

These and other embodiments will be further appreciated with respect to the following drawings, description, and examples.

### BRIEF DESCRIPTION OF THE SEVERAL VIEWS OF THE DRAWINGS

**Figure 1****.** X-ray diffraction graph of three batches of calcium sulfate hemihydrates.
**Figure 2****.** Percentage of weight loss of batches of calcium sulfate hemihydrates.
**Figure 3****.** Scanning Electro-Microscopy (SEM) images of Calcium sulfate hemihydrates.
**Figure 4****.** Scanning Electro-Microscopy (SEM) images of nHAC.
**Figure 5****.** The cure time of various mixing ratios of nHAC and calcium sulfate.
**Figure 6****.** Compressive strength of cured nHAC and calcium sulfate.
**Figure 7****.** Percentage weight loss from cured compositions with various Liquid (L) to Solid (S) ratios.
**Figure 8****.** In vitro degradation rate of cured nHAC and calcium sulfate.
**Figure 9****.** Cytocompatibility of cured nHAC and calcium sulfate.
**Figure 10****.** H&E staining for composition post implantation. (a) pure calcium sulfate and (b) 10% nHAC in the composition.

### DETAILED DESCRIPTION

The basic elements required for bone formation include a three-dimensional, open-porosity tissue scaffold, cells, and osteoinductive signaling molecules to stimulate cell differentiation, proliferation and matrix formation. Successful bone formation requires that these elements be combined in a well-coordinated spatial and time dependent fashion. The relative contribution of each element may vary, e.g., according to differences in patient age, gender, health, systemic conditions, habits, anatomical location, etc.

Embodiments for improved bone formation and healing include the following: biocompatible, open-porous bone tissues scaffold, enhanced local concentration of soluble bone mineral elements such as calcium and phosphate. Each is subsequently analyzed.

A biocompatible, open-porous bone tissue scaffold restores function and/or regenerates bone by providing a temporary matrix for cell proliferation and extracellular matrix deposition with consequent bone in-growth until new bony tissue is restored and/or regenerated. The matrix may also provide a template for vascularization of this tissue.

The macro and micro-structural properties of the scaffold influence the survival, signaling, growth, propagation, and reorganization of cells. They may also influence cellular gene expression and phenotype preservation. The following properties contribute to scaffold characteristics for bone formation: cell biocompatiability, surface chemistry, biodegradability, porosity, and pore size.

In one embodiment, the composition comprises mineralized collagen. Mineralized collagen is collagen matrix with hydroxyapatite particles dispersed in them in particular manner, simulating natural bone structure. Collagen is the main protein of connective tissue in animals and the most abundant protein in mammals. Bone is composed of strong, fibrillar bundles of collagen encased within a hard matrix of a calcium phosphate known as hydroxylapatite. Collagen is also a constituent in cartilage, tendon and other connective tissues.

Due to its high degree of biocompatibility with the human body, collagen has been successfully used in a variety of medical and dental applications for many years with minimal adverse responses. During its manufacture, potentially antigenic portions of the collagen molecule are removed, resulting in a product that is highly biocompatible and well-tolerated by the tissue. Collagen is also chemotactic for fibroblasts and other cells involved in bone tissue repair. Collagen biocompatibility ensures that the products are well integrated in the host tissue without eliciting an immune response.

Collagen used in the injectible composition may be from any source. These include natural sources such as human and mammalian tissues, and synthetic sources manufactured using recombinant technologies. It may be of any type (e.g., collagen Types I, II, III, X and/or gelatin). In one embodiment, collagen used is Type I collagen. In one embodiment, collagen is derived from bovine dermis. In one embodiment, fibrillar collagen is derived from bovine dermis manufactured by Kensey Nash Corporation (Exton Pa.) under the name Semed F. In one embodiment, the particles comprise at least about 33 percent by dry weight collagen. In another embodiment, the particles comprise from about 25 percent to about 75 percent dry weight collagen.

The surface chemistry of the scaffold can control and affect cellular adhesion. It can also influence the solubility and availability of proteins essential for intracellular signaling. Intracellular signaling maximizes osteoinductivity through controlled cellular differentiation, proliferation, and stimulation.

Collagen fabricates the disclosed structural scaffold and provides a physical and chemical milieu favorable to bone regeneration. Collagen also provides a favorable extracellular matrix for bone forming cells, e.g., osteoblasts, osteoclasts, osteocytes, etc. The bone forming cells' natural affinity for the collagen matrix has been demonstrated to favorably influence the function and signaling required for normal cellular activity.

The degradation rate of the scaffold should ideally match the bone-healing rate. Slower degradation rates can hinder the rate of remodeled, load-bearing bone formation. Faster degradation can result in unhealed defects.

The solubility and resorption of collagen is affected by its conformation and the degree of collagen cross-linking. The in vivo solubility and resorption of collagen is also influenced by the local concentration of proteolytic agents and vascularity at the site.

Scaffolds desirably posses an open pore, fully interconnected geometry to allow homogeneous and rapid cell in-growth, and facilitate vascularization of the construct from the surrounding tissue.

To this end, the total pore volume porosity of the scaffold simulates that of cancellous bone. Cancellous bone is a highly porous structure (about 50 vol. % to about 90 vol. %) arranged in a sponge-like form, with a honeycomb of branching bars, plates, and rods of various sizes called trabeculae. The synthetic scaffold must ensure pore interconnectivity to allow for the diffusion of nutrients and gases and for the removal of metabolic waste resulting from the activity of the cells within the scaffold. It is generally accepted by one skilled in the art that the pore diameters should be within the range of about 200 µm to about 900 µm range for ideal bone formation . Smaller pores can occlude and restrict cellular penetration, matrix production, and tissue vascularization. Larger pores can detrimentally influence the mechanical properties of the structural scaffold.

The disclosed method produces a synthetic scaffold that mimics the natural structural design of bone for bone formation. In one embodiment, the scaffold is fabricated using mineralized collagen. Mineralized collagen resembles the fundamental element of natural bone, allows the formation of a scaffold with high surface area and an interconnected network of high porosity. The disclosed composition and method supplements the local availability of essential soluble bone components, e.g., calcium and phosphate. Biologically compatible, sparingly soluble calcium phosphates are suitable supplements to locally increase the supply of soluble calcium [Ca²⁺] and phosphate [PO₄³⁻] ions

Bone growth factor cytokines, also known as bone morphogenetic proteins (BMPs), are entrapped at high concentration within bone and are secreted by many bone-forming cell types. The primary function of BMPs is cellular signaling. Intracellular signaling occurs through the binding of a soluble growth factor to a specific cell receptor site. This signal pathway stimulates several different and important bone healing events, including the proliferation, migration, and differentiation of bone forming cells. The cells are, in turn, responsible for the synthesis of other proteins and growth factors that are important for regulating and controlling bone tissue formation. Although there is a vast array of BMPs described and known to one skilled in the art, BMPs 2, 4, 6 and 7 are generally considered to be the most osteoinductive.

In one embodiment, the composition forms an injectible paste that enhances the formation of bone tissue. It is provided at a surgical site during reconstruction of a skeletal defect. For example, the injectible composition may be used in spine, dental, reconstructive, trauma, and other orthopedic surgeries. The injectible composition may be used as a substitute for or additive to autologous bone grafts. Although the composition is synthetic, it may include natural components, e.g., bovine collagen, and/or be combined with natural components, e.g., bone marrow aspirate.

In one embodiment, the injectible composition is both osteoinductive, i.e., it initiates or induces bone growth, and osteoconductive, i.e., it facilitates already initiated bone growth but does not itself initiate bone growth. Its osteoinductive effect arises, for example, from osteoinductive factors present in the liquid, e.g., bone marrow aspirate, used to make the paste.

A variety of calcium phosphate salts, represented by the general chemical formula xCaO,P₂0₅, may be used to simultaneously supplement the local [Ca²⁺] and [PO₄³⁻] ion concentrations and to act as short-term biologic buffers. In one embodiment, the composition includes a particulate mineralized collagen with calcium phosphate encased in collagen matrix.

In another embodiment, a method of making the particulate composition is provided. Mineralized Collagen combined with calcium sulfate, dried, crosslinked, and particulated as subsequently described.

In another embodiment, a method of using mineralized collagen and calcium sulfate particles is disclosed. The particulate composition can be combined with a fluid, for example, water, to create an injectible composition. The composition is then injected, manually applied, or otherwise delivered to a site of a bone. In one embodiment, the paste is an injectible bone void filler. The paste provides improved handling and delivery capabilities, allowing a surgeon to introduce the composition into complex geometry bone defects. The composition components are fully resorbable and stimulate bone regeneration in a manner similar to that achieved with natural bone.

In one embodiment, the composition contains particulate, mineralized collagen and calcium sulfate. The composition can be combined with a liquid such as biological fluids (e.g., bone marrow aspirate, whole blood, serum, plasma, etc.) to form an injectible paste. The paste is then used as an injectible and/or conformable (i.e., moldable) bone-grafting material for primary applications in, e.g., spine fusion, dental furcation augmentation, fracture repair, etc.

In one embodiment, where a collagen component is combined with a calcium phosphate component to produce a mineralized collagen component, porous particles of the mineralized collagen component may be prepared. In one embodiment, the mineralized collagen mixed with calcium sulfate in a ratio from about 0.5% to 50%. In another embodiment, the mineralized collagen mixed with calcium sulfate in a ratio from 5-30%.

In one embodiment, where a collagen component is combined with a calcium phosphate component to produce a mineralized collagen component, porous particles of the mineralized collagen component may be prepared. In one embodiment, the mineralized collagen mixed with calcium sulfate. The calcium sulfate might be calcium sulfate hemihydrates [CaSO₄-_{1/2}H₂O], partially dehydrate calcium sulfate [CaSO₄xH₂O, where x is between and includes 0 and 2]. The ratio of hemihydrate to dihydrate, ranging from 0.1% to 50%. In one embodiment, the ratios of hemihydrates to dehydrate, ranging from 2% to 25%.

A variety of calcium phosphate salts, represented by the general chemical formula Caₓ(PO₄)_{y}(O,OH,H₂O) may be used in the product composition to simultaneously supplement the local concentration of [Ca²⁺] and [PO₄³⁻] ion concentrations and to act as short-term biologic buffers. Calcium phosphates that may be used in the composition include monocalcium phosphate (monocal) [Ca(H₂PO₄)₂], calcium hydrogen phosphate (dical) [CaHPO_{4]}, calcium pyrophosphate [2CaO.P₂O₅], tricalcium phosphate [3CaO.P₂O₅], hydroxyapatite [3.33CaO.P₂O₅(OH)₂ (polycrystalline and amorphous compositions)], tetracalcium phosphate [4CaO-P₂O₅] and calcium carbonate [CaCO₃ (aragonite), CaCO₃ (calcite)]. In one embodiment, the composition comprises an acidic mixture of calcium phosphates. Acidic calcium phosphate refers to those compositions, with composite calcium (x)/phosphate (y) below 1.5, that either present acidic surface chemistries or solubilize in aqueous solution to a sufficient extent to cause solution buffering to an acidic value (pH<7.0). In one embodiment, the acidic calcium phosphate is calcium hydrogen phosphate dihydrate [CaHPO₄.2H₂O]. In one embodiment, the acidic calcium phosphate is anhydrous calcium hydrogen phosphate [CaHPO₄]. In one embodiment, the calcium phosphate of the composition is greater than about 25 percent by dry weight. In another embodiment, the calcium phosphate of the particulate composition is about 67 percent by dry weight.

The composition may further comprise additives such as bioactive agents, e.g., agents that exhibit biologic activity, and liquids. For example, agents that are osteoinductive and/or osteogenic may be included. As previously stated, osteoinductive agents stimulate bone growth. Examples of osteoinductive agents include bone growth factors, bone marrow components, blood components, and bone components. Bone growth factors may be purified or recombinant and include bone morphogenetic protein (BMP). Bone marrow aspirates (BMA) may be used in the composition because they contain osteoinductive agents such as bone growth factors and mesenchymal stem cells. Mesenchymal stem cells (MSCs) are multi-potent cells capable of differentiating along several lineage pathways to aid in the production of bone. MSCs are considered as a readily available source of cells for many tissue engineering and regenerative medicine applications. For these reasons, osteoinductive proteins and MSCs have been used to supplement the performance of osteoconductive bone formation scaffolds as replacements for autologous and allogeneic bone grafts.

Adding liquid to the composition results in an injectible composition, defined as soft masses with physical consistencies between a liquid and a solid. The liquid may be a biological fluid such as blood, plasma, serum, bone marrow, etc., or may be a buffer or may be capable of buffering to the physiological pH values of human serum (pH 7.1 to pH 7.4). Examples of buffers are known to one skilled in the art and include Tris and phosphate-buffered saline. In one embodiment, the composition has a pH in the range of about pH 5 to about pH 7.4. In another embodiment, the composition has a pH in the range of about pH 5.5 to about pH 6.9. More than one liquid may be included in the composition. For example, the composition may include bone marrow aspirate and a buffering salt solution. The liquid may also include biocompatible liquids such as water, saline, glycerin, surfactants, carboxylic acids, dimethylsulfoxide, and/or tetrahydrofuran. In one embodiment, the liquid is greater than about 25 percent by volume of the composition. In another embodiment, the liquid comprises from about 30 percent to about 55 percent by volume of the composition. Additionally, natural and synthetic polymers such aliphatic polyesters, polyethylene glycols, polyanhydrides, dextran polymers, derivatized above mentioned polymers, and/or polymeric orthophosphates may be included in the composition.

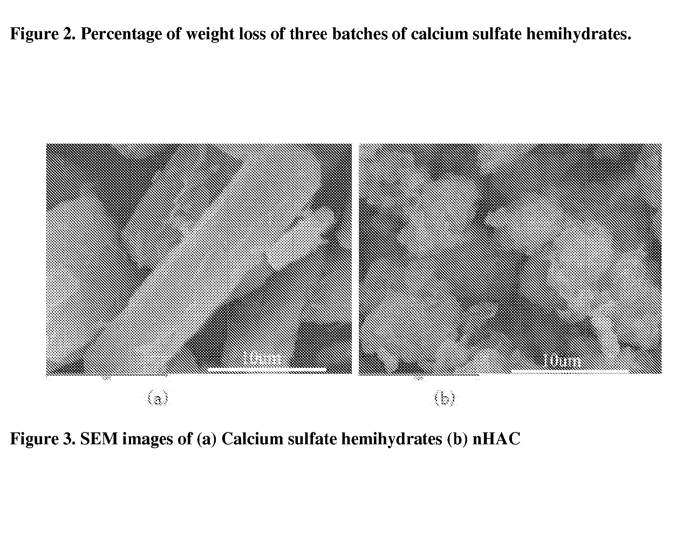

In one embodiment, a process for producing a particulate mineralized collagen composition comprising collagen and calcium sulfate is provided. In one embodiment, a mineralized collagen and calcium sulfate composition is prepared and is then formed into particles, as shown in FIG. 3. The types of collagen that may be used are described above and include bovine dermal collagen. Suitable calcium phosphate includes acidic calcium phosphate such as monocalcium phosphate [Ca(H₂PO₄)₂], calcium hydrogen phosphate [CaHPO₄], and/or calcium pyrophosphate [2CaO.P₂O₅]. Mineralized collagen then can be further processed by freezing, lyophilization, and the solid composition is formed into particles. Methods of forming particles are known to one skilled in the art and include, but are not limited to, grinding, milling, chopping, and/or molding. In one embodiment, particles are formed by milling the solid composition. Milling may occur using a Wiley mill (Thomas Scientific, Swedesboro N.J.). The mesh size on the mill directs the size of the resultant particles. In one embodiment, a -20 mesh is used that creates particles in the range of about 100 µm to about 840 µm. The particles may be sized by, for example, sieving. At any point in the process, additional components may be added to the composition, as described above. For example, an osteoinductive component can be added prior to forming the articles. Upon combining the mineralized collagen with calcium sulfate, the composition may be provided as a kit. In one embodiment, the kit includes the composition described above, and may further include other components. These include a receptacle such as a plastic container in which to place the composition and in which to add liquid to form the composition into a paste or putty, a mixing implement such as a spatula, stir rod, etc., a disposable syringe barrel without a needle in which to place and deliver the mixed paste, instructions for formulating and/or using the composition, etc.

In another embodiment, a method of facilitating bone growth is provided. In one embodiment, the method includes adding at least one osteoinductive component such as a purified bone growth factor, a recombinant bone growth factor, a bone marrow component, a blood component, demineralized bone, autologous bone, etc., to the particulate composition previously described. In embodiments where the osteoinductive component is bone marrow aspirate, blood, or a blood component, it may be acutely obtained and added to the composition (e.g., blood and/or bone marrow may be obtained from the same surgical site for repairing the defect). Adding the osteoinductive component(s) and/or another liquid to the composition, with stirring, results in a paste or putty, which is provided to the desired anatomical site of the patient.

In one embodiment, the paste is loaded into the barrel of a disposable 5 cc syringe, without a needle attached, and is extruded through the barrel aperture to the desired anatomical site. In another embodiment, the putty is manipulated or formed into a configuration of desired size, shape, length, etc., either manually or by instrumentation, and gently pressed on and/or in the desired anatomical site. The site is desirably prepared to expose healthy bleeding bone, facilitating subsequent bone growth. The method may be performed using minimally invasive procedures known to one skilled in the art. The method may be used in at least partially filling bone voids and/or gaps of the skeletal system (i.e., extremities, pelvis, spine, oral cavity) that are not intrinsic to the stability of the bone structure. These voids and/or gaps may be a result of trauma, either natural or by surgical creation. The paste is gently provided on and/or in the void and/or gap. The paste is resorbed by the body during the healing process (over days, weeks, and months). The paste may be molded into the bone void or defect by manipulating either manually or using an instrument (e.g., spatula, syringe, probe, cannula, etc.).

The following examples further illustrate embodiments of the invention.

### EXAMPLE 1: Preparation of calcium sulfate hemihydrate (CaSO₄•1/2H₂O)

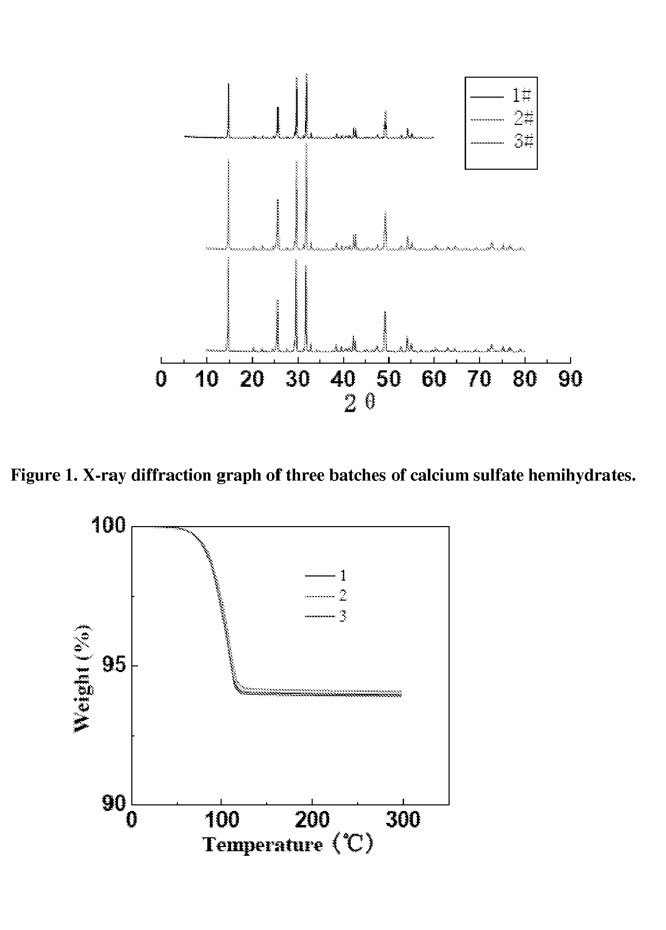

Calcium sulfate dihydrate was purchased from Merck Co. (Whitehouse station, NJ, USA). Sodium citrate, aluminum sulfate was purchased from Sigma Aldrich (St. Louis, MO, USA). Calcium sulfate hemihydrate was prepared from Calcium sulfate dehydrate using the thermal dehydration method. Briefly, 300 grams of Calcium sulfate dihydrate was added into the reactor, 0.75 grams of sodium citrate and 0.75 grams of aluminum sulfate were also added into the reactor. 1701 grams of deionized water was added into the reactor. The solution was mixed at 400 rpm at 120°C for 6 hours. The reactant mixture was poured into a beaker to be filtered. The process was repeated and rinsed for 5 times. The filtrate (CaSO₄•1/2H₂O) was dried at 100ₒ C overnight. The dried powder was further sieved by 100 µm sieve to get powder of uniform sizes. X-ray diffraction (XRD) analysis was done on a X-ray diffractor 08Discovery (Siemens, Germany). The XRD results were shown in Figure 1. The XRD results indicated the major diffraction peaks at 14.752, 25.576, 29.756, 31.936, 49.212 and 54.232 degrees were the same as the XRD patterns obtained from the calcium sulfate hemihydrate USTA standard, proving the formation of calcium sulfate hemihydrates.

Thermal Gravity Analysis (TGA) was done on TGA2050 (TA Instrument Corporation, USA) at heating rate of 10 °C/min. The percent of weight loss from three batches of preparations was shown in Figure 2. The weight loss from three batches was 1) 6.04%; 2) 5.91% and 3) 6.08%, close to the theoretical value for calcium sulfate hemihydrate : 6.2%.

### EXAMPLE 2: Preparation of mineralized collagen and calcium sulfate hemihydrate (CaSO₄•1/2H₂O) compositions.

Calcium sulfate hemihydrates was prepared as described in example 1. Mineralized collagen (nHAC) was obtained from Beijing Allgens Medical Technology Limited (Beijing, China).

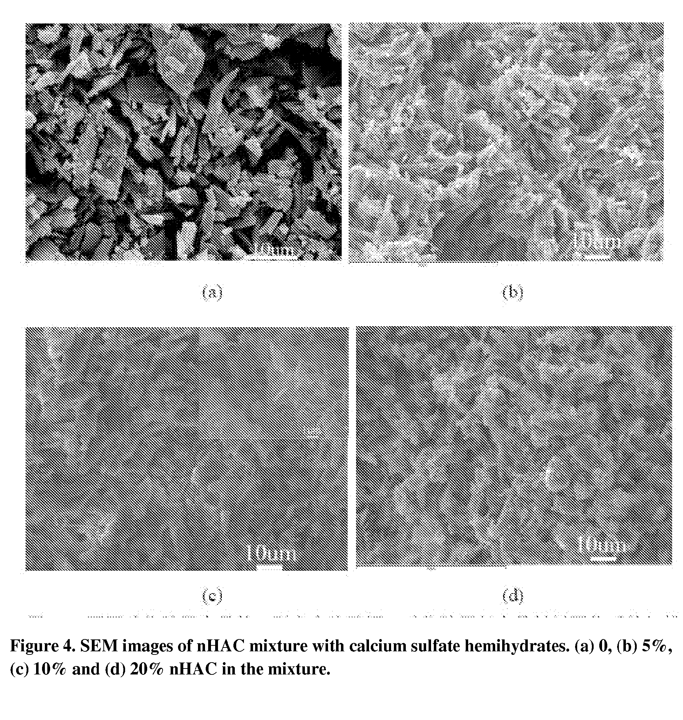

Physical mixture of Calcium sulfate hemihydrates and mineralized collagen (nHAC) was prepared by mixing the two powders. Morphology of the mixture was examined on Scanning Electromicroscopy (SEM) on JSM-6460LV (Joel, Japan) and results were shown in Figure 3 and Figure 4.

Various ratios of calcium sulfate dihydrate may also be included into the mineralized collagen and calcium sulfate hemihydrates compositions to shorten the curing time of the putty.

### EXAMPLE 3: The injectibility and mechanical integrity of injectible composition of mineralized collagen (nHAC) and calcium sulfate mixture.

Various ratios of nHAC and calcium sulfate was mixed very uniformly. The nHAC ratios ranged from 0, 5%, 10% and 20%. The solid mixture was further mixed with water in the ratio of solid to liquid from 0.5 to 1. The injectibility of the composition was tested by loading the formulation into a 5mL syringe (Beck and Dickenson, Franklin lakes, NJ, USA). The injectibility was classified using the following table 1.

**Table 1. Criteria for injectibility of nHAC and calcium sulfate composition using a 5mL syringe.**

| Injectibility | Description |
|---|---|
| Superior | Very easy to inject without exerting much force |
| Good | Easy to inject with exerting force with ease |
| Fair | Inject with force, but extrudate not continuous |
| Poor | Inject with great force, frequent stop during the injection due to resistance. |
| Not injectible | Not passing through the syringe at all |

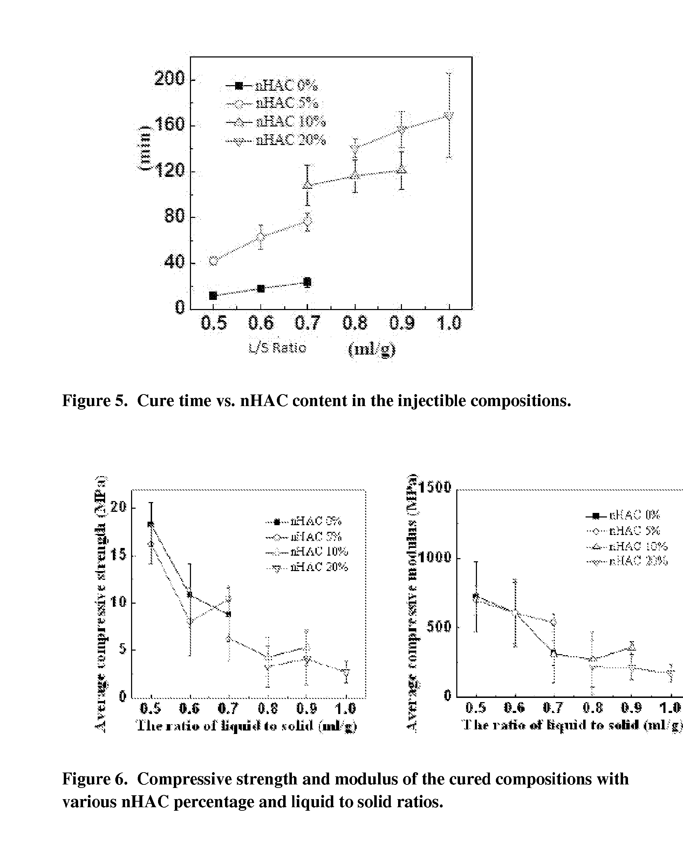

The cure time of the initially flowable (injectible) composition was tested on an electroforce mechanical test station (Shanghai BONTE mechanical test instrument corporation, China) using standardized test method: IS09597-1989E. The initial curing time was taken as the dial insert (280 g, Φ1.13 mm) into the solution at distance to the test base of 5 ± 1 mm, and the final curing time was taken as the dial insert not able to insert into the cured composition. The results of various mixing ratios of nHAC and calcium sulfate were shown in Figure 5.

### EXAMPLE 4: The mechanical properties of the cured composition of mineralized collagen (nHAC) and calcium sulfate mixture.

Cured nHAC and calcium sulfate composition was prepared into a TEFLON mold of dimensions of 6 mm in diameter and 10 mm in height. The cylinder was tested on an INSTRON mechanical tester for its compression properties. The compression rate was 1 mm/min. Tests were done on three replicates and mean was reported in Figure 6.

### EXAMPLE 5: The in-vitro degradation of the cured mineralized collagen (nHAC) and calcium sulfate mixture.

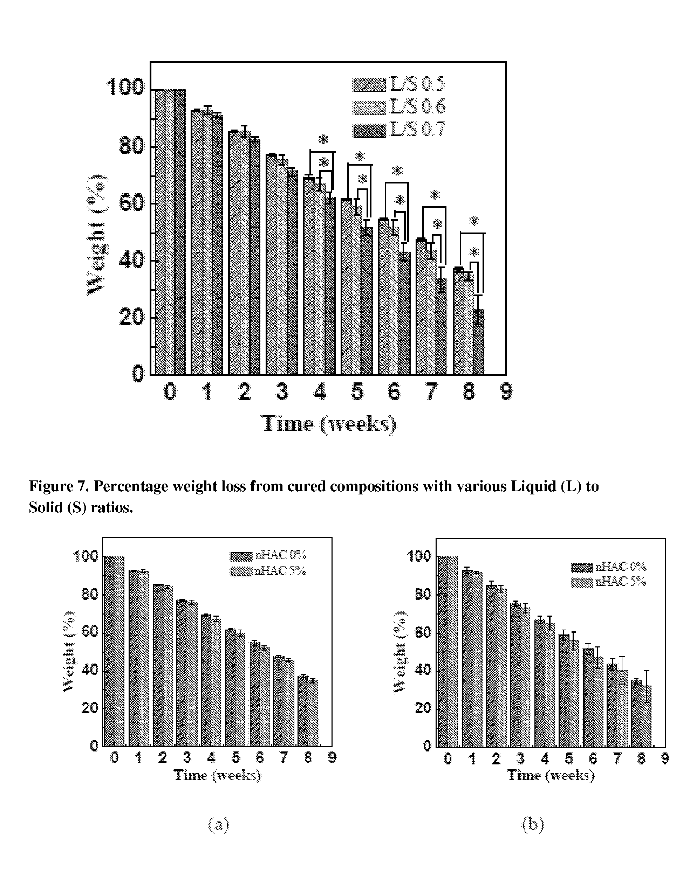

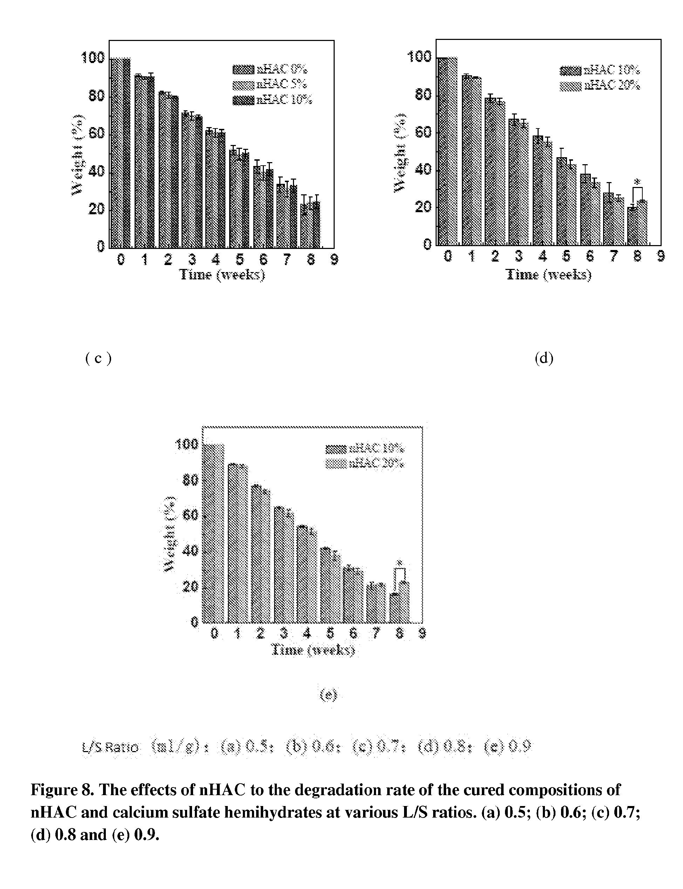

In vitro degradation behavior of the cured cylinder described in example 4 was tested as follows: cylinders were placed into 5 ml of simulated body fluid at 37ₒC. The in-vitro degradation set up was placed in a shaker which rotated at 60 rpm. The simulated body fluid was replaced every two days and fresh simulated body fluid was replenished. At 1, 2, 4 and 8 weeks into the experiment, the weight of the cylinder was recorded to reflect the weight loss. Percentage weight changes were taken as the degradation rate. Results of percentage weight loss in relation to the L/S ration were shown in Figure 7. The results of percentage weight loss in relation to the nHAC were shown in Figure 8.

### EXAMPLE 6: Cytocompatibility of the cured mineralized collagen (nHAC) and calcium sulfate mixture.

Each 1 mL of cells was mixed with 10 mL of alpha minimum essential medium (α-MEM) in the presence of 10% fetal bovine serum (FBS) and 1% penicillin-streptomycin (PS). The cell suspension was plated in a cell culture dish and incubated under 37°C, 5% CO2 environment. When the concentration of the MC3T3-E1 osteoblastic cells reached 3 x 10⁵ cells/mL, they were seeded onto the experimental substrate of interest (nHAC and calcium sulfate), which were then placed on a 12-well polystyrene plate, and stored in a CO₂ incubator for 2, 4, or 6 days to observe cell morphology and count viable attached cells as a function of incubation time. The concentration of the cells seeded onto the specimen substrate was 1.0 x 10⁵ cells/well.

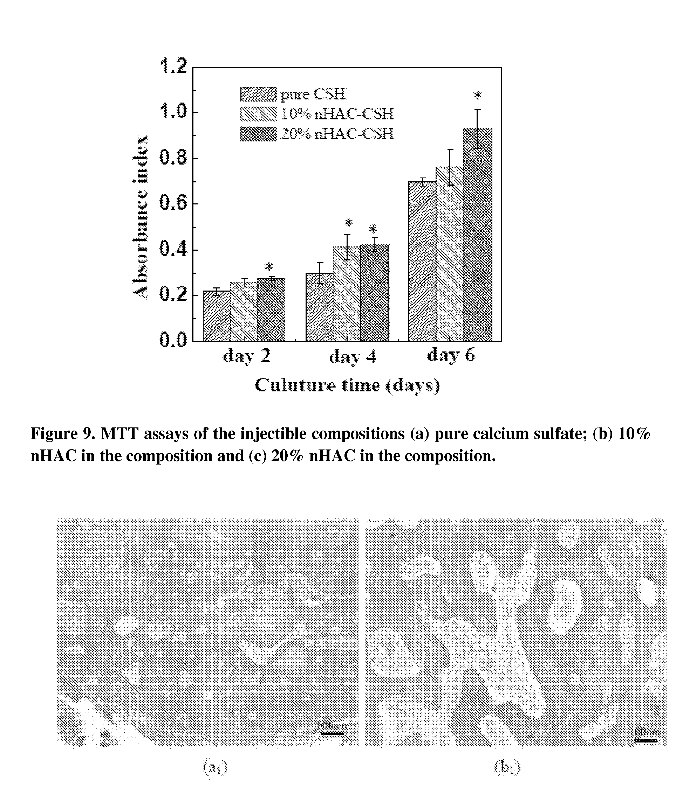

To estimate the density of viable cells, MTT assay was employed. After the selected incubation periods, the samples were washed by PBS and transferred to a new 12-well polystyrene culture plate. MTT dye agent (1 mL; Sigma, St. Louis, MO) was added to each well. After 3 h of incubation in 5% CO₂ incubator, 1 mL of isopropanol was added to each well and the polypstyrene plate was shaken for 30 min. After waiting for 30 min, the absorbance of each solution was measured at the wavelength of 570 nm with the subtraction of the 650 nm background by spectrophotometer (Biomate3, Thermo Electron, Madison, WI). The MTT results were shown if Figure 9.

### Example 7: Implantation and Bone healing study of the cured mineralized collagen (nHAC) and calcium sulfate (CS) mixture.

Prior to opening a container containing particles of the above composition, the volume of a bone void to be repaired was determined. Based on the bone void, an appropriate volume of water (liquid) was obtained, using a L/S ratio of 0.75:1 were added, as subsequently described, to obtain products of desired cohesive consistency (e.g. paste). As one example, per 1 gram of dry particle, 0.5 ml water was added to obtain a cohesive putty, or 0.85 ml water was added to obtain a paste. Other liquid phase might also include blood, bone marrow aspirtes, etc. As another example, per 1 cc dry particle volume, 0.75 ml bone marrow aspirate was added to obtain a cohesive putty, or 0.85 ml bone marrow aspirate was added to obtain a paste.

Immediately prior to implantation on an isolated bone, the liquid was mixed with the composition to obtain a paste of desired consistency. The bone void site was irrigated as needed and the paste was packed into the bone void. The site was sealed with surrounding soft tissue as needed, e.g., to close the wound and restore soft tissue configuration. Rigid fixation of the defect site stabilized the bone void.

It should be understood that the embodiments and examples described are only illustrative and are not limiting in any way. Therefore, various changes, modifications or alterations to these embodiments may be made or resorted to without departing from the spirit of the invention and the scope of the following claims.

For the animal study we used 24 female New Zealand White Rabbits. Under general anaesthesia a critical size defect of 4,0 mm diameter and 10 mm in depth was drilled into the metaphysis of distal femoral condyles perpendicular to the long axis of the bone. The pure calcium sulfate and the calcium sulfate (10% nHAC) paste were implanted at the defect sits. In our animal model the host bone surrounding the implants undergoes physiological strain during unrestricted movement of the animals. For evaluation of bone formation and implant change with time animals had been sacrificed after, 4, 8 and 12 weeks. The cylindrical defects were filled with a total volume of 0,125 ml of the implant paste. Each animal received two randomly distributed implants into the distal femoral condyles. After sacrificing the animals the thigh bone was dissected from the surrounding soft tissue and processed for histological staining. The bones underwent a procedure with fixation in ethanol with increasing concentrations (40%-100%), two times defatting with Roticlear^{®} and finally embedding in polymethylmethacrylate (PMMA) resin containing a softening agent and stepwise enlarging concentrations of an accelerator. After polymerisation the bone samples were sliced perpendicular to the long axis by diamond cutting. Subsequently the specimens had been grinded and polished up to a final thickness of about 50 µm and stained with H&E's staining for examination in conventional light microscopy. Using a Zeiss Axioplan 2 microscope we evaluated the bone formation in a region including the drill whole and a rim of adjacent host bone. The fate of the implant itself was examined in light microscopy and Environmental Scanning Electron Microscopy (ESEM) delivering more detailed informations of the implant and its interface with surrounding bone tissue

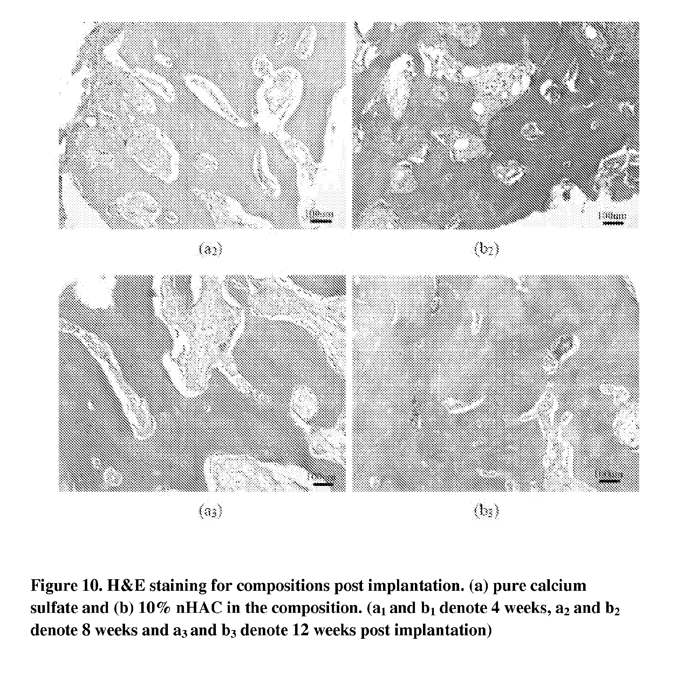

All surgical incisions healed uneventfully without infection or wound dehiscence. Generally the nHAC group heals bone defects more efficiently than the pure CS group: In the major part of animals the implants were resorbed faster for the CS group at all survival times (Figure 10 b₁, b₂ and b₃). CS group degraded faster than the nHAC containing group (Figure 10 a₁, a₂ and a₃).

## Claims

1. A process for producing a bone growth composition comprising combining a mineralized collagen component with a calcium sulfate component to produce an injectible composition, wherein the mineralized collagen component comprises the form of a sponge prior to creating the particles, wherein creation of the particles includes milling.

2. The process of claim 1 wherein the calcium sulfate component comprises the form of calcium sulfate hemihydrates.

3. The process of claim 1 comprising adding an osteoinductive component to the composition.

4. The process of claim 3 wherein the osteoinductive component is added after the particles are formed.

5. The process of claim 1 wherein combining a mineralized collagen component with a calcium sulfate dihydrate component to produce an injectible composition.

6. The composition of claim 5 further comprising lyophilizing the mineralized collagen component.

7. The process of claim 1 wherein the mineral in the mineralized collagen is selected from the group consisting of monocalcium phosphate [Ca(H₂PO₄)₂], calcium phosphate dibasic [CaHPO_{4]}, calcium pyrophosphate [2CaO•P₂O₅], hydraxyapatite and combinations thereof.

8. The process of claim 1 wherein the ratios of mineralized collagen to calcium sulfate are from 0.5% to 35%.

9. The process of claim 1 wherein calcium sulfate hemihydrate is hydrothermally dehydrated.

10. A process for producing a bone growth composition comprising combining a mineralized collagen with a calcium sulfate component, and creating particles of the mineralized collagen component, wherein the mineralized collagen component comprises the form of a sponge prior to creating the particles, further comprising including the particles in a kit.

11. The process of claim 10 wherein the kit includes a device for mixing the particles with a fluid.

12. The process of claim 10 wherein the kit includes a receptacle for mixing the particles with a fluid.

13. A process for producing a bone growth composition comprising combining a mineralized collagen component with a calcium sulfate component to produce a injectible composition, and creating particles of the mineralized collagen component, wherein the mineralized collagen component comprises the form of a sponge prior to creating the particles, further comprising sizing the particles.

14. The process of claim 13 wherein the size of the particles is selected by sieving.
